# EUROPEAN PATENT APPLICATION

(11) **EP 3 202 315 A1**
(43) Date of publication of application: **09.08.2017**
(21) Application number: 16782919.1
(22) Date of filing: 23.03.2016
(51) Int. Cl.: A61B 5/0285

(54) **MEDICAL DEVICE AND OPERATING METHOD FOR MEDICAL DEVICE**

(30) Priority: 21.04.2015 JP 2015086977
(71) Applicant: Olympus Corporation, Hachioji-shi, Tokyo 192-8507 (JP)
(72) Inventor: IGARASHI, Makoto, Hachioji-shi, Tokyo 192-8507 (JP); WATANABE, Takeshi, Hachioji-shi, Tokyo 192-8507 (JP); HIRAI, Yuji, Hachioji-shi, Tokyo 192-8507 (JP); KIMURA, Kenichi, Hachioji-shi, Tokyo 192-8507 (JP); HOMMA, Satoshi, Hachioji-shi, Tokyo 192-8507 (JP); MIYAJIMA, Hiroshi, Hachioji-shi, Tokyo 192-8507 (JP)
(74) Representative: Gunzelmann, Rainer
(86) International application number: PCT/JP2016/059154
(87) International publication number: WO 2016/170897

(57) **Abstract**

A medical apparatus includes a laser light source for blood vessel sensing (31) configured to irradiate illumination light for detecting a blood vessel, a photodetector (33) configured to detect return light of the illumination light from a subject, an optical-characteristic processing circuit (41) configured to calculate, based on a detection result of the photodetector (33), at least one of information concerning scattering of blood cells in the blood vessel and information concerning absorption by blood in the blood vessel, a blood-vessel-characteristic determination circuit (42) configured to determine a characteristic of the blood vessel based on a calculation result of the optical-characteristic processing circuit (41), and a laser light source for guide (32) configured to perform notification based on the characteristic of the blood vessel determined by the blood-vessel-characteristic determination circuit (42).

## Description

### Technical Field

The present invention relates to a medical apparatus that acquires information related to a blood vessel from return light of illumination light and an operating method for the medical apparatus.

### Background Art

For example, in a laparoscopic operation, it is a basic user need to avoid intraoperative bleeding (there is a significant risk in proceeding with dissection in a blind manner while being unable to be aware of presence of a blood vessel). However, in a state in which the blood vessel cannot be properly gripped, even a device having an excellent hemostasis ability cannot exhibit a sufficient blood vessel sealing ability. Therefore, under a situation in which blood vessel running is unclear, it is necessary to carefully proceed with detachment of a tissue.

In the following explanation, several clinical examples are explained.

A transverse colon has a lot of veins and an extremely diverse blood vessel running variation. In a transverse colon operation, hemostasis is difficult once bleeding occurs. It takes time to stanch the bleeding. Therefore, in the operation, the point is to make it possible to dissect the transverse colon without causing bleeding around the transverse colon.

In a portion where a fat tissue is thick in a mesentery, it is difficult to identify a blood vessel. In an operation, a delicate manipulation for identifying a blood vessel is performed by carefully bringing a device (including an energy device) into contact with fat and moving the device and acutely or obtusely detaching the fat to expose the blood vessel. Therefore, it is important from a viewpoint of securing safety to check, under an observation visual field of an endoscope, how a relatively thick blood vessel such as a middle colic artery, an inferior mesenteric artery, or a branching blood vessel from these arteries runs.

As it is seen from these examples, it greatly contributes to safety and simplification of an operation to make it possible to visually recognize a blood vessel.

Therefore, a device mounted with a function of sensing a blood vessel with light such as laser has been disclosed.

For example, International Publication No. 2013/134411 discloses a device mounted with a function of sensing a blood vessel with light such as laser. More specifically, International Publication No. 2013/134411 mentions that, for example, the device includes a laser Doppler velocimeter, the device generates an alarm signal when detecting a blood vessel having a diameter larger than a threshold, the device generates alarm display, an LED at a device distal end can perform lighting/flash light emission/a color change, and the device stops an energy applicator (performs an off-control in on/off control) according to a sensing value.

It is desirable to acquire not only the information described in International Publication No. 2013/134411 but also more detailed information concerning the blood vessel and further improve safety and simplicity.

The present invention has been devised in view of the circumstances and an object of the present invention is to provide a medical apparatus that can acquire more detailed information concerning a blood vessel and improve safety and simplicity of medical services.

### Disclosure of Invention

### Means for Solving the Problem

A medical apparatus according to a certain aspect of the present invention includes: an illuminating section connected to an insertion object, which is inserted into a body cavity of a subject, and configured to irradiate illumination light for detecting a blood vessel of the subject; a detecting section configured to detect return light from the body cavity of the subject of the illumination light irradiated by the illuminating section; a calculating section configured to calculate, based on a detection result of the detecting section, at least one of information concerning scattering of blood cells in the blood vessel and information concerning absorption by blood in the blood vessel; a determining section configured to determine characteristics of the blood vessel based on a calculation result of the calculating section; and a notifying section configured to perform notification based on the characteristics of the blood vessel determined by the determining section.

An operating method for a medical apparatus according to a certain aspect of the present invention includes: a step in which an illuminating section connected to an insertion object, which is inserted into a body cavity of a subject, irradiates illumination light for detecting a blood vessel of the subject; a step in which a detecting section detects return light from the body cavity of the subject of the illumination light irradiated by the illuminating section; a step in which a calculating section calculates, based on a detection result of the detecting section, at least one of information concerning scattering of blood cells in the blood vessel and information concerning absorption by blood in the blood vessel; a step in which a determining section determines characteristics of the blood vessel based on a calculation result of the calculating section; and a step in which a notifying section performs notification based on the characteristics of the blood vessel determined by the determining section.

### Brief Description of the Drawings

Fig. 1 is a diagram showing a configuration example of a medical apparatus in an embodiment of the present invention;
Fig. 2 is a perspective view showing a configuration of a distal end portion of a dissection device in the embodiment of the present invention;
Fig. 3 is a flowchart showing action of the medical apparatus in the embodiment of the present invention;
Fig. 4 is a graph showing a state in which a frequency spectrum temporally fluctuates according to a pulsation in the embodiment of the present invention;
Fig. 5 is a graph showing an example in which the frequency spectrum is different depending on thickness of a blood vessel in the embodiment of the present invention;
Fig. 6 is a graph showing an example in which the frequency spectrum is different depending on depth of the blood vessel in the embodiment of the present invention;
Fig. 7 is a diagram showing a disposition example of operation members related to determination of thickness of a blood vessel near a handle section of the dissection device in the embodiment of the present invention;
Fig. 8 is a diagram showing a disposition example of operation members related to determination of an artery/a vein near the handle section of the dissection device in the embodiment of the present invention;
Fig. 9 is a graph showing a state in which an average frequency spectrum is different depending whether a blood vessel is only an artery, only a vein, or the artery and the vein in the embodiment of the present invention;
Fig. 10 is a graph showing an example of temporal fluctuation of a frequency spectrum of the artery due to a pulsation in the embodiment of the present invention;
Fig. 11 is a graph showing light absorbances at a time when the vein and the artery are present in the embodiment of the present invention;
Fig. 12 is a graph comparing and showing light absorbances at a time when only the artery is present and at a time when the vein and the artery are present in the embodiment of the present invention;
Fig. 13 is a graph showing wavelength dependencies of absorption spectra of venous blood and arterial blood together with wavelength dependencies of absorption spectra of hemoglobin Hb and hemoglobin dioxide HbO2 in the embodiment of the present invention;
Fig. 14 is a diagram showing an example of monitor display at a time when the artery (or parallel running of the artery and the vein) is detected and, for example, green guide light is irradiated on a subject from a laser light source for guide in the embodiment of the present invention;
Fig. 15 is a diagram showing an example of monitor display at a time when the vein is detected and blue guide light is irradiated on the subject from the laser light source for guide in the embodiment of the present invention;
Fig. 16 is a diagram showing a configuration example in which a laser light source of a three-wave irradiation type is used as a laser light source for blood vessel sensing in the embodiment of the present invention;
Fig. 17 is a diagram showing an example in which operation members for setting depth of a target blood vessel are disposed near the handle section of the dissection device in the embodiment of the present invention;
Fig. 18 is a diagram showing an example in which the green guide light is irradiated on the artery, the blue guide light is irradiated on the vein, and chroma of blue or green is set higher as thickness of the blood vessel is thicker in the embodiment of the present invention;
Fig. 19 is a left side view showing an example in which images obtained by irradiating the green guide light a plurality of times along the artery and irradiating the blue guide light a plurality of times along the vein are superimposed and the artery and the vein are displayed in the embodiment of the present invention;
Fig. 20 is a front view showing the example in which the images obtained by irradiating the green guide light a plurality of times along the artery and irradiating the blue guide light a plurality of times along the vein are superimposed and the artery and the vein are displayed in the embodiment of the present invention;
Fig. 21 is a plan view showing the example in which the images obtained by irradiating the green guide light a plurality of times along the artery and irradiating the blue guide light a plurality of times along the vein are superimposed and the artery and the vein are displayed in the embodiment of the present invention;
Fig. 22 is a diagram showing an example in which a region of arteriosclerosis emerges in the blood vessel because of cholesterol or the like and flow velocity of a blood flow changes in the embodiment of the present invention;
Fig. 23 is a diagram showing an example in which an arteriosclerosis portion where the blood flow is relatively fast and a portion other than the arteriosclerosis where the blood flow is relatively slow are color-coded by guide lights having different colors in the embodiment of the present invention;
Fig. 24 is a diagram showing a state in which cerebral aneurysm occurs in a cerebral artery and a blood flow occurs in the cerebral aneurysm in the embodiment of the present invention;
Fig. 25 is a diagram showing an example in which a cerebral aneurysm portion where the blood flow is relatively slow and a portion other than the cerebral aneurysm where the blood flow is relatively fast are color-coded by the guide lights having different colors in the embodiment of the present invention;
Fig. 26 is a diagram showing a state in which clipping treatment is performed on the cerebral aneurysm and the blood flow in the cerebral aneurysm is stopped in the embodiment of the present invention;
Fig. 27 is a diagram showing an example of color-coding by the guide lights of the cerebral aneurysm portion where the blood flow is stopped by the clipping treatment and the portion other than the cerebral aneurysm where the blood flow is relatively fast in the embodiment of the present invention;
Fig. 28 is a diagram showing a state in which the clipping treatment is performed on the cerebral artery other than the cerebral aneurysm and a portion where the blood flow is hindered emerges in the cerebral artery in the embodiment of the present invention;
Fig. 29 is a diagram showing an example of color-coding by the guide lights of the cerebral aneurysm portion where the blood flow is stopped by the clipping treatment and the cerebral artery in which the blood flow is hindered and the cerebral artery in which the blood flow is relatively fast and is not hindered in the embodiment of the present invention;
Fig. 30 is a diagram showing a state in which the blood flow remains in the cerebral aneurysm because the clipping treatment on the cerebral aneurysm is insufficient in the embodiment of the present invention;
Fig. 31 is a diagram showing an example of color-coding by the guide lights of the cerebral aneurysm portion where the slow blood flow remains because of the insufficient clipping treatment and the portion other than the cerebral aneurysm where the blood flow is relatively fast in the embodiment of the present invention;
Fig. 32 is a diagram showing an example of disposition of operation members for emitting laser light for sensing near the handle section of the dissection device in the embodiment of the present invention;
Fig. 33 is a diagram showing an example in which the laser light for sensing is outputted in an axial direction from the distal end portion of the dissection device in the embodiment of the present invention; and
Fig. 34 is a diagram showing an example in which the laser light for sensing is outputted from a lower jaw toward an upper jaw of the distal end portion of the dissection device in the embodiment of the present invention.

### Best Mode for Carrying Out the Invention

An embodiment of the present invention is explained below with reference to the drawings.

### [Embodiment]

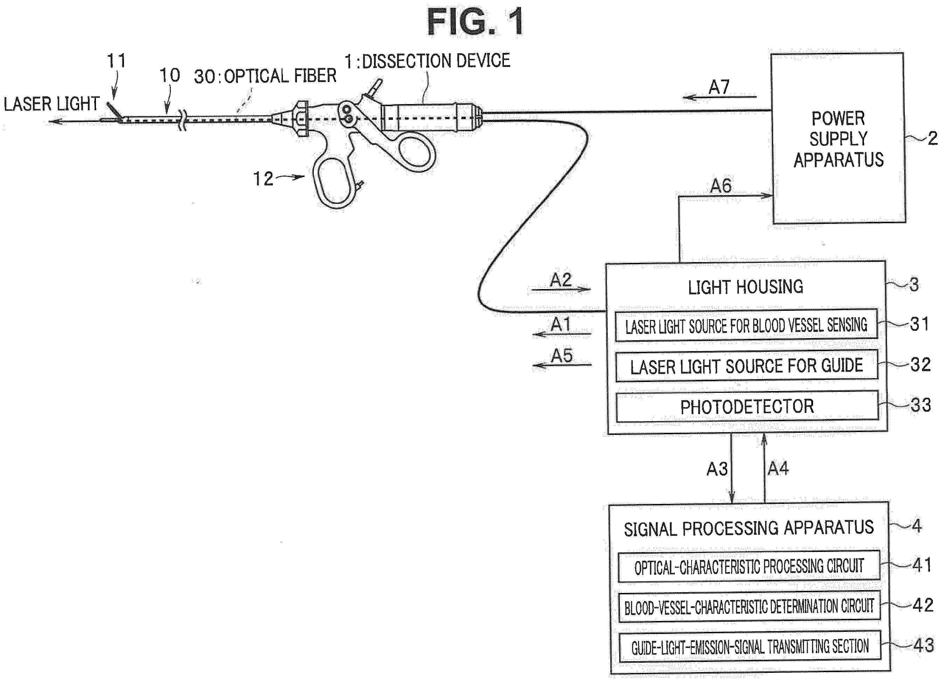

Fig. 1 to Fig. 34 show an embodiment of the present invention.

### [Medical apparatus]

First, a medical apparatus is explained with reference to Fig. 1 to Fig. 6.

Fig. 1 is a diagram showing a configuration example of the medical apparatus.

The medical apparatus in the present embodiment includes, for example, a dissection device 1, a power supply apparatus 2, a light housing 3, and a signal processing apparatus 4.

The dissection device 1 is an insertion object inserted into a body cavity of a subject and is a treatment device (an energy device) configured to apply energy of heat, light, an electric current, or the like to the subject and capable of dissecting a tissue of the subject. The dissection device 1 further includes, according to necessity, a treatment function (e.g., a sealing function by resistance heating energy) for sealing the subject. The dissection device 1 is desirably a treatment device capable of operating in a plurality of operation modes (operation modes such as highfrequency cauterization (HF) and ultrasound coagulation dissection (US)) in which blood vessel sealing abilities are different.

The dissection device 1 includes an insertion section 10 inserted into the subject, a treatment section 11 provided at a distal end portion of the insertion section 10, and a handle section 12 for operating the dissection device 1. Further, an optical fiber 30 is inserted through the dissection device 1 from a proximal end side toward a distal end portion on the treatment section 11 side.

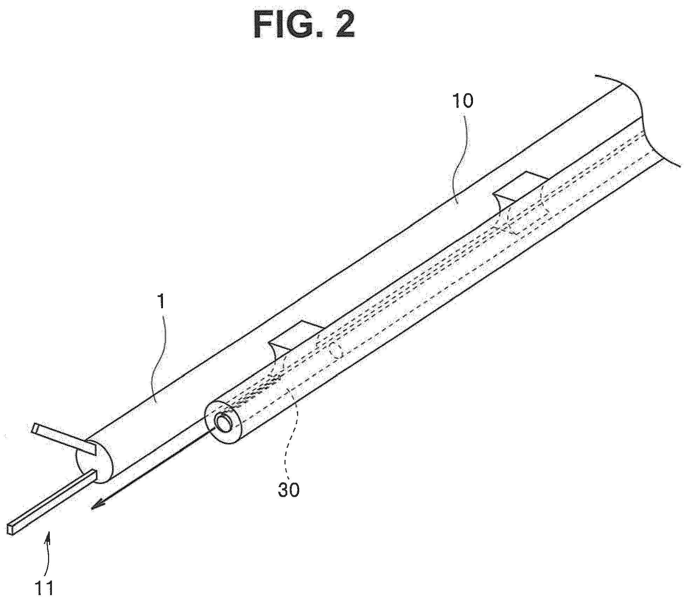

Fig. 2 is a perspective view showing a configuration of a distal end portion of the dissection device 1.

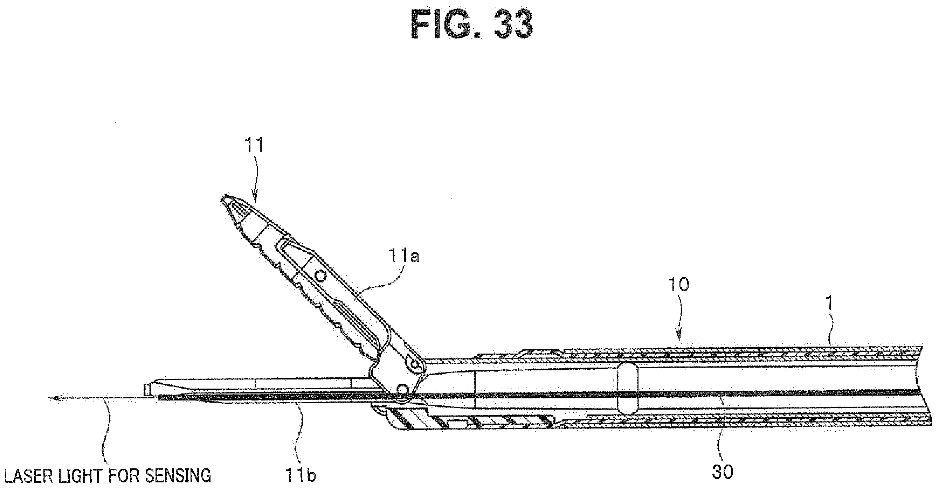

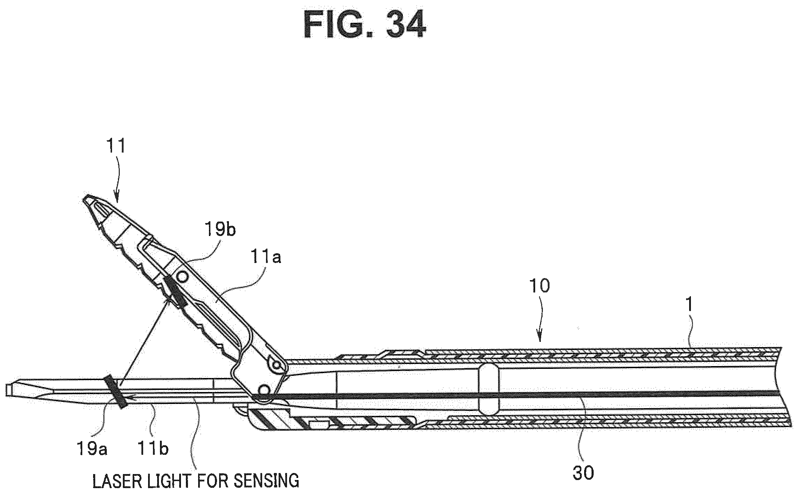

At the distal end portion of the insertion section 10 of the dissection device 1, for example, the treatment section 11 including an upper jaw 11a and a lower jaw 11b (see, for example, Fig. 33 and Fig. 34) for holding and treating the subject is provided. The optical fiber 30 is disposed near the treatment section 11 such that a distal end is exposed (i.e., laser light can be irradiated toward the subject).

The power supply apparatus 2 supplies energy for treatment to the dissection device 1.

A proximal end side of the optical fiber 30 explained above is connected to the light housing 3. The light housing 3 supplies laser light to the optical fiber 30. More specifically, the light housing 3 includes a laser light source for blood vessel sensing 31, a laser light source for guide 32, and a photodetector 33.

The laser light source for blood vessel sensing 31 is an illuminating section configured to irradiate illumination light (laser light for sensing) for detecting a blood vessel of the subject. The laser light source for blood vessel sensing 31 is configured as a semiconductor light source apparatus configured to generate laser light having at least one kind of a wavelength. The generated laser light is irradiated on the subject from a distal end of the optical fiber 30 (therefore, the distal end portion of the dissection device 1 where the distal end of the optical fiber 30 is disposed) as laser light for sensing for blood vessel sensing.

The laser light source for guide 32 is a notifying section configured to perform notification based on characteristics of a blood vessel determined by a blood-vessel-characteristic determination circuit 42, which is a determining section explained below. More specifically, the laser light source for guide 32 generates laser light for guide (referred to as guide light as appropriate) serving as notification light for performing color coding guide corresponding to the characteristics of the blood vessel. The guide light is also irradiated on the subject via the optical fiber 30. Therefore, the laser light source for guide 32 also functions as a light emitting section capable of selectively generating notification light (e.g., blue light) having a first wavelength to be irradiated on an irradiation near region including an irradiation region on which illumination light is irradiated in the subject and notification light (e.g., green light) having a second wavelength different from the first wavelength to be irradiated on the irradiation near region.

The photodetector 33 is a detecting section configured to detect return light (reflected light) from the body cavity of the subject of the laser light for sensing irradiated by the laser light source for blood vessel sensing 31, which is the illuminating section.

The signal processing apparatus 4 analyzes the laser light for sensing irradiated by the light housing 3 and the return light from the subject, estimates characteristics of a blood vessel of the subject, and further controls, according to the estimated characteristics of the blood vessel, the guide light irradiated by the light housing 3. More specifically, the signal processing apparatus 4 includes an optical-characteristic processing circuit 41, the blood-vessel-characteristic determination circuit 42, and a guide-light-emission-signal transmitting section 43.

The optical-characteristic processing circuit 41 is a calculating section configured to calculate, based on a detection signal outputted from the photodetector 33, at least one of information concerning scattering (a Doppler frequency spectrum (hereinafter simply referred to as frequency spectrum) concerning information such as blood flow velocity and a pulsation) of blood cells (more specifically, red blood cells containing hemoglobin) in the blood vessel and information concerning absorption by blood in the blood vessel.

More specifically, the optical-characteristic processing circuit 41 is capable of calculating information concerning flow velocity of flowing of the blood cells in the blood vessel by performing a frequency analysis of the detection signal outputted from the photodetector 33 to calculate a frequency spectrum and calculating an area in the frequency spectrum (an integrated value of intensity of the frequency spectrum), a gradient of the frequency spectrum, or an average frequency of the frequency spectrum.

The blood-vessel-characteristic determination circuit 42 is a determining section configured to determine presence or absence of a blood vessel based on the information calculated by the optical-characteristic processing circuit 41 and, when determining that a blood vessel is present, determine at least one of thickness of the blood vessel, whether the blood vessel is an artery or a vein, and depth of the blood vessel as characteristics of the blood vessel (a property or the like of the blood vessel).

Note that an information acquiring section configured to detect a spectrum of return light from the subject of the illumination light irradiated by the illuminating section and acquire, based on the spectrum, as characteristics of a blood vessel in an irradiation region where the illumination light is irradiated on the subject, at least one of thickness information of the blood vessel, depth information of the blood vessel, and type information of the blood vessel indicating whether the blood vessel is the artery or the vein includes the photodetector 33, the optical-characteristic processing circuit 41, and the blood-vessel-characteristic determination circuit 42.

The guide-light-emission-signal transmitting section 43 is a control section configured to control the light emitting section to emit the notification light having the first wavelength as a first notification method when the blood characteristic determined by the blood-vessel-characteristic determination circuit 42, which is the determining section, is a first characteristic and control the light emitting section to emit the notification light having the second wavelength as a second notification method different from the first notification method when the blood characteristic determined by the blood-vessel-characteristic determination circuit 42 is the second characteristic different from the first characteristic. More specifically, the guide-light-emission-signal transmitting section 43 transmits, to the laser light source for guide 32 of the light housing 3, according to the characteristic of the blood vessel determined by the blood-vessel-characteristic determination circuit 42, a trigger signal for emitting the guide light.

Note that, in the following explanation, it is assumed that, besides the components shown in Fig. 1, an endoscope such as a laparoscope, which is an insertion object inserted into the body cavity of the subject, a monitor for observing an endoscopic image and various kinds of information, and the like are further disposed in order to observe the subject.

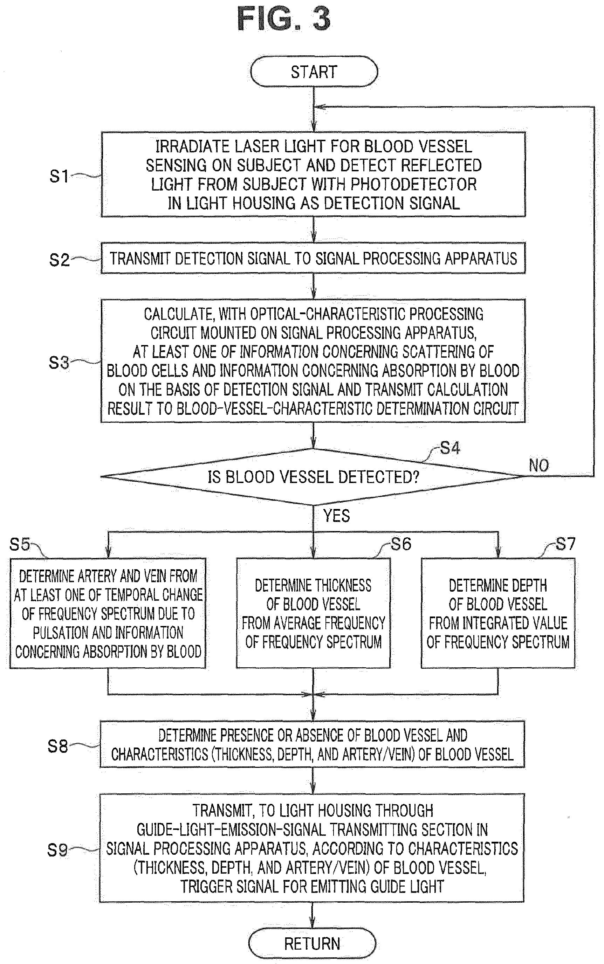

Fig. 3 is a flowchart showing action of the medical apparatus.

When entering processing shown in the flowchart from not-shown main control processing, the medical apparatus emits laser light for sensing from the laser light source for blood vessel sensing 31 and, as indicated by an arrow A1 in Fig. 1, transmits the laser light for sensing to the distal end side of the dissection device 1 via the optical fiber 30 and irradiates the laser light for sensing toward the subject. Further, the medical apparatus receives, from the distal end of the optical fiber 30, return light from the subject, transmits the return light to the light housing 3 as indicated by an arrow A2 via the optical fiber 30, and performs detection in the photodetector 33 to generate a detection signal (step S1).

The detection signal generated by the photodetector 33 is transmitted from the light housing 3 to the signal processing apparatus 4 as indicated by an arrow A3 (step S2).

In the signal processing apparatus 4 that receives the detection signal, the optical-characteristic processing circuit 41 calculates, based on the detection signal, at least one of information concerning scattering (a frequency spectrum concerning information such as blood flow velocity and a pulsation) of blood cells (more specifically, red blood cells containing hemoglobin) in a blood vessel and information concerning absorption by blood in the blood vessel and transmits a calculation result to the blood-vessel-characteristic determination circuit 42 (step S3).

The blood-vessel-characteristic determination circuit 42 determines based on the information received from the optical-characteristic processing circuit 41 whether the blood vessel is detected (step S4).

When the blood vessel is not detected, the signal processing apparatus 4 transmits information indicating that the blood vessel is not detected to the light housing 3 as indicated by an arrow A4. Consequently, the processing returns to step S1 explained above. The light housing 3 emits laser light for sensing from the laser light source for blood vessel sensing 31, transmits the laser light for sensing to the distal end side of the dissection device 1 via the optical fiber 30 as indicated by an arrow A5, and irradiates the laser light for sensing toward the subject. The signal processing apparatus 4 continuously performs the detection of the blood vessel as explained above.

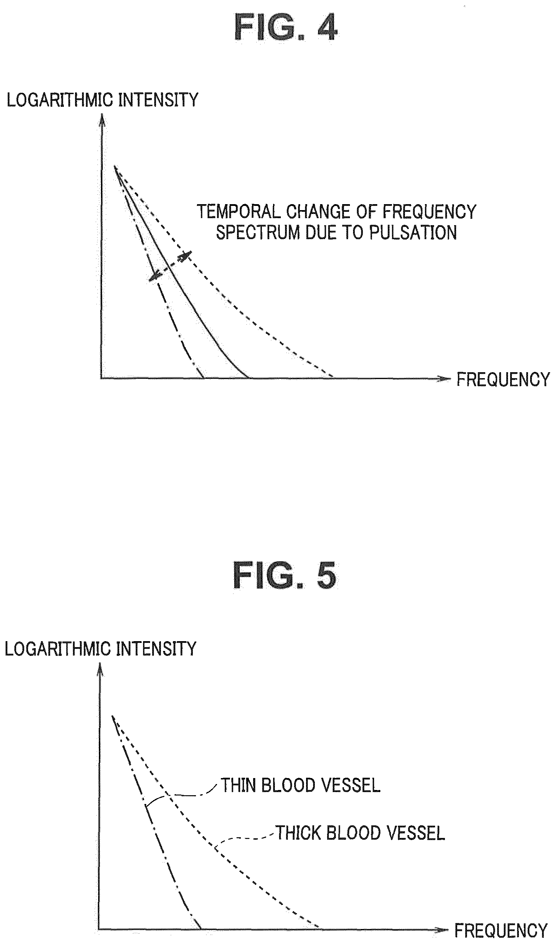

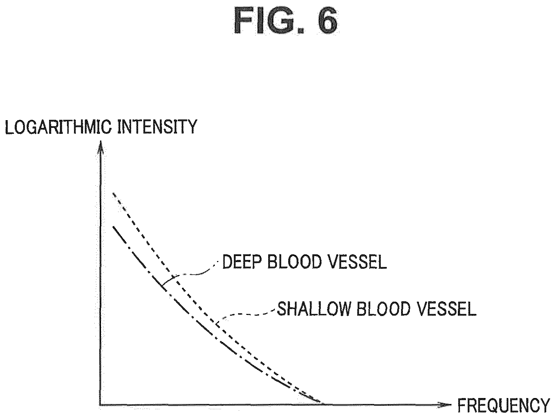

When the blood vessel is detected in step S4, the blood-vessel-characteristic determination circuit 42 further executes at least one of determining based on at least one of temporal fluctuation of the frequency spectrum due to the pulsation (see Fig. 4) and the information concerning absorption by the blood whether the blood vessel is the artery or the vein (step S5), determining thickness of the blood vessel from an average frequency of the frequency spectrum (see Fig. 5) (step S6), and determining depth of the blood vessel from an integrated value of the frequency spectrum (see Fig. 6) (step S7).

Fig. 4 is a graph showing a state in which the frequency spectrum temporally fluctuates because of the pulsation. Fig. 5 is a graph showing an example in which the frequency spectrum is different depending on thickness of the blood vessel. Fig. 6 is a graph showing an example in which the frequency spectrum is different depending on depth of the blood vessel.

As shown in Fig. 4, when the blood vessel is the artery, the frequency spectrum temporally changes. On the other hand, when the blood vessel is the vein, a conspicuous temporal change of the frequency spectrum in the artery does not occur. Therefore, it is possible to determine whether the blood vessel is the artery or the vein based on presence or absence of a temporal change of the frequency spectrum.

Therefore, the signal processing apparatus 4 functions as a processor including an input section to which information indicating a frequency spectrum of light from the subject including the blood vessel is inputted, a fluctuation monitoring section configured to monitor temporal fluctuation of the information indicating the frequency spectrum inputted to the input section and, when the temporal fluctuation exceeds a predetermined threshold, detect the temporal fluctuation as an excess over the threshold, and an output section configured to, when the excess over the threshold is detected by the fluctuation monitoring section, output information indicating that the blood vessel is the artery and, when the excess over the threshold is not detected, output information indicating that the blood vessel is the vein.

As shown in Fig. 5, since blood flow velocity is low when the blood vessel is thin, logarithmic intensity of the frequency spectrum rapidly decreases as a frequency increases. However, since the blood flow velocity is high when the blood vessel is thick, the logarithmic intensity of the frequency spectrum decreases at a more gentle degree according to the frequency. Therefore, the thickness of the blood vessel is determined by calculating, for example, an average of the frequency spectrum.

Further, as shown in Fig. 6, the logarithmic intensity of the frequency spectrum is higher at the same frequency in a blood vessel having small depth than a blood vessel having large depth. Therefore, an integrated value of the frequency spectrum is calculated to determine depth of the blood vessel.

In this way, the blood-vessel-characteristic determination circuit 42 determines based on results of the respective determinations whether the blood vessel is present in an irradiation region of the laser light for sensing and characteristics (thickness, depth, and the artery/the vein) of the blood vessel (step S8).

Further, the guide-light-emission-signal transmitting section 43 transmits, to the laser light source for guide 32 of the light housing 3, according to the characteristics (the thickness, the depth, and the artery/the vein) of the blood vessel determined by the blood-vessel-characteristic determination circuit 42, a trigger signal for emitting the guide light (note that the trigger signal is not transmitted when it is determined that the blood vessel is absent) as indicated by the arrow A4. Consequently, the guide light corresponding to the characteristics of the blood vessel is emitted from the laser light source for guide 32 and irradiated on the blood vessel set at a target (a target blood vessel) (step S9). Further, the light housing 3 receives a determination result of the characteristics of the blood vessel from the signal processing apparatus and outputs a control signal indicated by an arrow A6 to the power supply apparatus 2. Consequently, the power supply apparatus 2 changes electric power outputted to the dissection device 1 to electric power corresponding to the characteristics of the blood vessel as indicated by an arrow A7.

Thereafter, the medical apparatus returns to the not-shown main control processing. When continuously performing the blood vessel sensing, the medical apparatus enters the processing again from the main control processing.

### [Determination of thickness of the blood vessel]

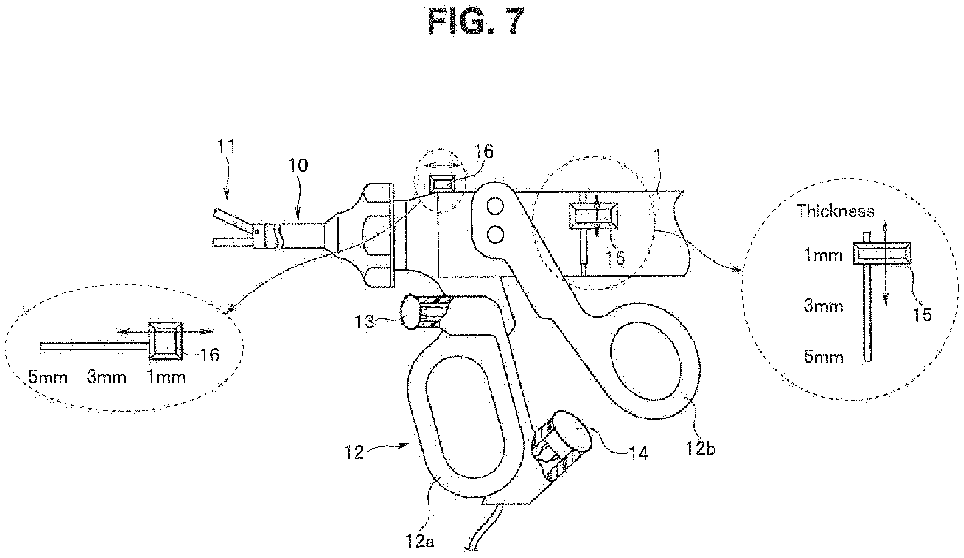

Fig. 7 is a diagram showing a disposition example of operation members related to determination of thickness of the blood vessel near the handle section 12 of the dissection device 1.

The handle section 12 is an operation section configured to perform operation for dissecting the tissue of the subject. The handle section 12 has structure in which a first handle 12a and a second handle 12b respectively including finger rings are opened and closed by hinge mechanisms and operated. A first button section 13 is provided on an inserting direction side of a proximal end portion of the first handle 12a. A second button section 14 is provided on the second handle 12b side of a distal end portion of the first handle 12a. A first slide section 15 is provided on a side surface further on a hand side than the second handle 12b of a main body that supports the first handle 12a and the second handle 12b. A second slide section 16 is provided on an upper surface of the main body that supports the first handle 12a and the second handle 12b.

The first button section 13, the second button section 14, the first slide section 15, and the second slide section 16 are provided in positions where the first button section 13, the second button section 14, the first slide section 15, and the second slide section 16 can be operated by a hand that operates the handle section 12. Not all of the first button section 13, the second button section 14, the first slide section 15, and the second slide section 16 need to be provided. At least one of the first button section 13, the second button section 14, the first slide section 15, and the second slide section 16 only has to be provided. The first button section 13, the second button section 14, the first slide section 15, and the second slide section 16 are used as, for example, operation sections configured to set thickness of the target blood vessel.

In this case, for example, in the first slide section 15 and the second slide section 16, indicators "1 mm", "3 mm", and "5 mm" indicating thicknesses of blood vessels are described together with characters "Thickness" according to necessity. For example, the medical apparatus is set such that the first slide section 15 is slid up and down with a thumb of a surgeon (or the second slide section 16 is slid back and forth with an index finger of the surgeon) and aligned with a position of any one of the indicators, whereby a blood vessel having thickness designated by the indicator (or a blood vessel having thickness equal to or larger than the thickness (a threshold of a blood vessel diameter)) designated by the indicator is sensed.

The surgeon may press the first button section 13 and the second button section 14 to detect a blood vessel having predetermined thickness.

Therefore, the first button section 13, the second button section 14, the first slide section 15, and the second slide section 16 function as a threshold switching section disposed in the handle section 12, which is the operation section, and capable of changing a threshold of thickness information of a blood vessel acquired by the information acquiring section.

When the guide light is irradiated on the blood vessel, the thickness of which is detected as explained above, by the laser light source for guide 32 to perform guide display, for example, the guide display is performed as explained below.

When the blood vessel is thick (e.g., a diameter is equal to or larger than 2 mm), green guide light is irradiated on the subject. When the blood vessel is thin (e.g., a diameter is smaller than 2 mm), blue guide light is irradiated on the subject. Only when the blood vessel is thick, the green guide light may be irradiated on the subject or the green guide light may be flashed and irradiated on the subject to perform flashing display.

Further, a light emission ratio of a green laser light source and a blue laser light source in the laser light source for guide 32 may be changed to perform color coding display corresponding to the thickness of the blood vessel. As a specific example, the green guide light is irradiated on a thick blood vessel, bluish green guide light is irradiated on a blood vessel having intermediate thickness, and the blue guide light is irradiated on a thin blood vessel to perform the color coding display.

In addition, in association with switching of the target blood vessel from a certain blood vessel to another blood vessel by operation of the handle section 12 or the like, settings for, for example, changing a threshold for obtaining a signal processing result, changing the number of times and determination steps of integration processing in signal processing (e.g., the number of times of integration and the determination steps are increased when the target blood vessel is thin), and increasing/reducing to change, according to the signal processing result, electric power (e.g., a laser light source output) supplied from the power supply apparatus 2 to the dissection device 1 are updated in the medical apparatus as a whole.

When the blood vessel is thick, a risk during bleeding is higher than when the blood vessel is thin. Therefore, when the dissection device 1 includes both of a blood vessel sealing ability and a dissection ability, it is desirable to automatically set the power supply apparatus 2 in a mode for achieving both of the blood vessel sealing ability and the dissection ability when a thick blood vessel is detected and automatically set the power supply apparatus 2 in a mode for regarding the dissection ability as important when a thin blood vessel is detected or a blood vessel is not detected. In this case, at least one of the signal processing apparatus 4 and the light housing 3 functions as a control section configured to perform control to switch an operation mode of the treatment device according to the characteristics of the blood vessel determined by the blood-vessel-characteristic determination circuit 42, which is the determining section. Consequently, it is possible to perform effective treatment without depending on determination of the surgeon.

### [Determination of a type (the artery/the vein) of the blood vessel]

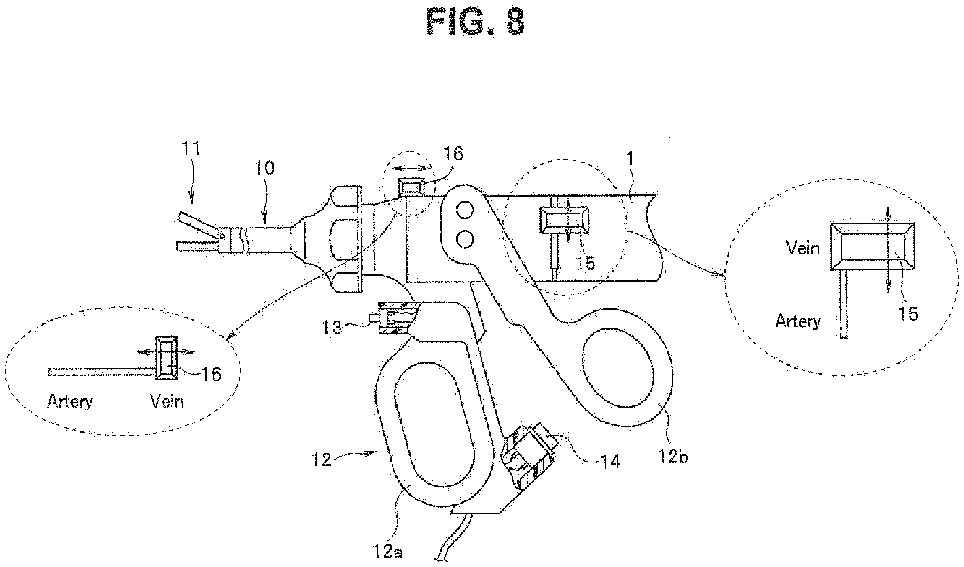

Determination of a type (the artery/the vein) of the blood vessel is explained with reference to Fig. 8 to Fig. 15.

Fig. 8 is a diagram showing a disposition example of operation members related to determination of the artery/the vein near the handle section 12 of the dissection device 1.

Concerning the determination of the artery/the vein, for example, the first slide section 15 and the second slide section 16 are used as operation members for setting thresholds of characteristics of the target blood vessel. The first button section 13 and the second button section 14 are used for generation of a trigger signal for power application by the power supply apparatus 2 (however, at least one of the first button section 13 and the second button section 14 may be used as the operation members for setting the thresholds of the characteristics of the target blood vessel). Therefore, as the operation members for setting thresholds of characteristics of the target blood vessel, it is unnecessary to provide both of the first slide section 15 and the second slide section 16. At least one of the first slide section 15 and the second slide section 16 only has to be provided.

In this case, indicators "Vein" and "Artery" indicating types of blood vessels are described in, for example, the first slide section 15 and the second slide section 16. For example, the medical apparatus is set such that the first slide section 15 is slid up and down with the thumb of the surgeon (or the second slide section 16 is slid back and forth with the index finger of the surgeon) and aligned with a position of any one of the indicators, whereby a blood vessel of a type designated by the indicator is sensed. When the first slide section 15 or the second slide section 16 is slid to a position of "Vein", a detection target is set as the vein. When the first slide section 15 or the second slide section 16 is slid to a position of "Artery", the detection target is set as the artery (or parallel running of the artery and the vein). Therefore, blood vessels other than the set detection target are excluded from the detection target.

In this way, the first slide section 15 and the second slide section 16 function as a threshold switching section disposed in the handle section 12, which is the operation section, and capable of changing a threshold of type information of a blood vessel acquired by the information acquiring section.

As running states of the target blood vessel, there are three patterns of (1) running as the artery alone, (2) running as the vein alone, and (3) parallel running of the artery and the vein.

Therefore, not only detecting (1) or (2), for example, an indicator "Vein + Artery" may be added between the indicators "Vein" and "Artery" to detect only (3).

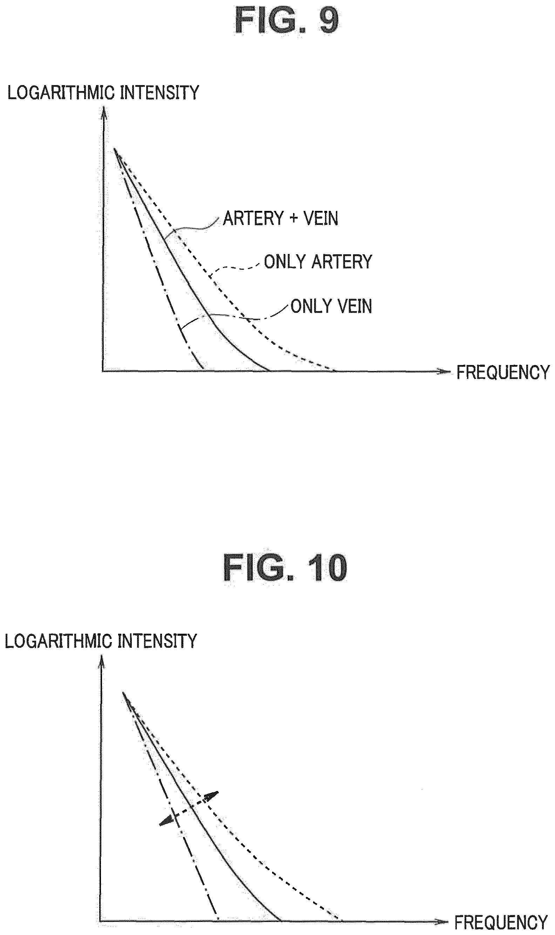

Fig. 9 is a graph showing a state in which an average frequency spectrum is different depending on whether the blood vessel is only the artery, only the vein, or the artery and the vein.

In an average frequency of a frequency spectrum, as shown in Fig. 9, a relation of the vein alone<the parallel running of the artery and the vein<the artery alone occurs. Therefore, it is possible to determine based on the average frequency whether the target blood vessel is only the artery, only the vein, or the artery and the vein. Electric power supplied from the power supply apparatus 2 to the dissection device 1 is changed based on a determination result.

On the other hand, when it is sufficient to determine whether the detection target is the artery (or the parallel running of the artery and the vein) or only the vein, the determination is performed, for example, as shown in Fig. 10.

Fig. 10 is a graph showing an example of temporal fluctuation of the frequency spectrum of the artery due to a pulsation.

The determination concerning whether the target blood vessel is the artery (or the parallel running of the artery and the vein) or the vein is performed based on whether there is temporal change of the frequency spectrum due to a pulsation. In the artery (or the parallel running of the artery and the vein), as shown in Fig. 10, the frequency spectrum fluctuates up and down in association with a pulsation. On the other hand, since the vein does not have a pulsation, up-down fluctuation of the frequency spectrum due to a pulsation does not occur.

Therefore, it is possible to detect temporal fluctuation of the frequency spectrum, when the temporal fluctuation is detected, determine that the blood vessel is the artery (or the parallel running of the artery and the vein), and, when the temporal fluctuation is not detected, determine that the blood vessel is the vein. More specifically, the blood-vessel-characteristic determination circuit 42 calculates, for example, an average frequency, when the calculated average frequency temporally fluctuates, determines that the blood vessel is the artery (or the parallel running of the artery and the vein), and, when the average frequency does not fluctuate, determines that the blood vessel is the vein.

Note that the determination of the artery (or the parallel running of the artery and the vein) and the vein may be performed based on a temporal fluctuation characteristic of light absorbance due to a pulsation.

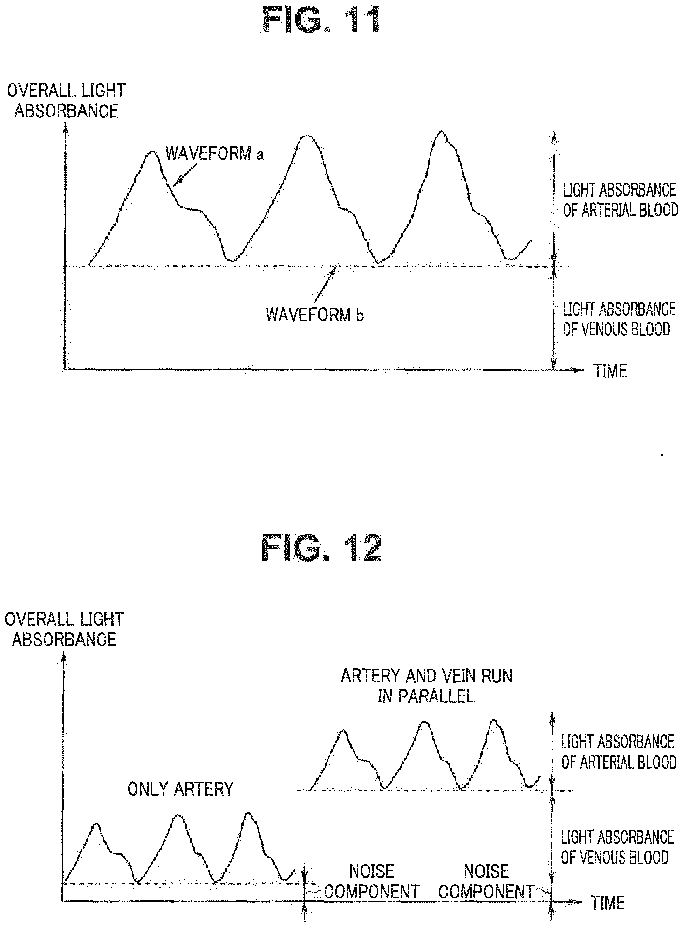

Fig. 11 is a graph showing light absorbances at a time when the vein and the artery are present.

In the artery, the light absorbance temporally fluctuates because a volume of a region where light is irradiated changes according to a pulsation (see a waveform "a" in Fig. 11). However, in the vein, the light absorbance does not temporally fluctuate because there is no pulsation (see a waveform "b" in Fig. 11).

Therefore, when the waveform "a" concerning the light absorbance is obtained, it is possible to determine that the target blood vessel is the artery alone or the parallel running of the artery and the vein. When the waveform "b" concerning the light absorbance is obtained, it is possible to determine that the target blood vessel is the vein alone.

Note that it is possible to determine based on height of a baseline of a graph indicating a time characteristic of the light absorbance whether the target blood vessel is the artery alone or the parallel running of the artery and the vein.

Fig. 12 is a graph comparing and showing light absorbances at a time when only the artery is present and at a time when the vein and the artery are present.

When the light absorbance at the time when the target blood vessel is the artery alone and the light absorbance at the time when the target blood vessel is the parallel running of the artery and the vein are compared, as shown in Fig. 12, the baseline is higher by light absorbance of venous blood when the target blood vessel is the parallel running of the artery and the vein than when the target blood vessel is the artery alone (note that noise components are superimposed on both of the light absorbances). Therefore, the artery alone or the parallel running of the artery and the vein only have to be determined based on the height of the baseline.

Further, it is also possible to determine the artery and the vein based on spectrum reflected light intensity of return light from the subject.

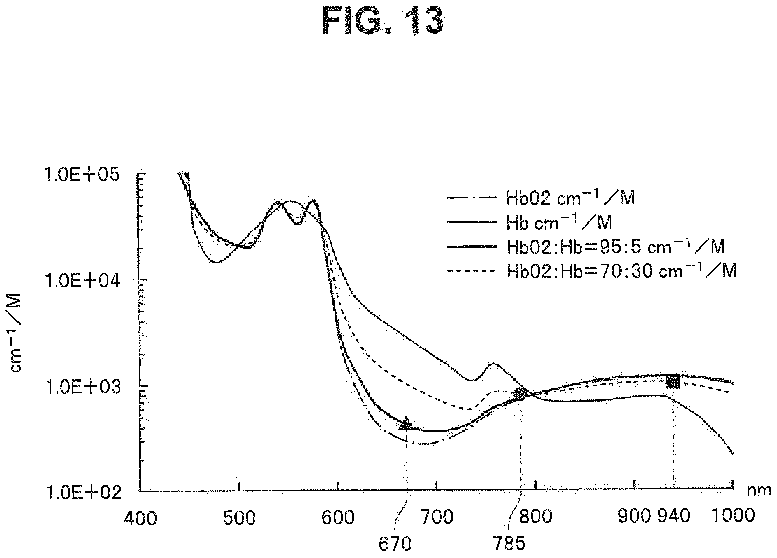

Fig. 13 is a graph showing wavelength dependencies of absorption spectra of venous blood and arterial blood together with wavelength dependencies of absorption spectra of hemoglobin Hb and hemoglobin dioxide HbO2.

In Fig. 13, a ratio of HbO2 and Hb of the arterial blood is modeled as being HbO2:Hb = 95:5 and a ratio of HbO2 and Hb of the venous blood is modeled as being HbO2:Hb = 70:30 and the wavelength dependencies of the absorption spectra are shown.

In order to perform a spectrum analysis, it is necessary to change a wavelength of the laser light for sensing to multiple wavelengths. Therefore, in this case, the laser light source for blood vessel sensing 31 is configured to include three kinds of semiconductor laser light sources that respectively irradiate, for example, laser lights for sensing having three kinds of wavelengths of 670 nm, 785 nm, and 940 nm.

In this case, as shown in Fig. 13, in the arterial blood, light absorption of the laser light for sensing having the wavelength of 670 nm is lower than light absorption of the laser light for sensing having the other wavelengths (785 nm and 940 nm). Further, an absorption characteristic of the laser light for sensing having the wavelength of 785 nm is substantially equal in the artery and the vein. However, an absorption characteristic of the laser light for sensing having the wavelength of 670 nm is larger in the vein compared with the artery.

Therefore, for example, first, laser Doppler detection is performed by the laser light for sensing having the wavelength of 785 nm to determine whether the blood vessel is present.

When determining that the blood vessel is present, further, the laser light for sensing having the wavelength of 670 nm, for example, is irradiated. A ratio I (670 nm)/I (785 nm) of reflected light intensity I (785 nm) detected at the wavelength of 785 nm and reflected light intensity I (670 nm) detected at the wavelength of 670 nm is calculated.

When the ratio I (670 nm)/I (785 nm) calculated in this way is larger than a predetermined threshold, it is determined that the target blood vessel is the artery.

Note that the laser light for sensing having the wavelength of 670 nm is used. However, instead of the laser light for sensing having the wavelength of 670 nm, the laser light for sensing having the wavelength of 940 nm may be used. In this case, an absorption characteristic of the laser light for sensing having the wavelength of 940 nm is small in the vein compared with the artery. Therefore, it is sufficient to determine that the target blood vessel is the artery when a ratio I (940 nm)/I (785 nm) of the reflected light intensity is smaller than other predetermined thresholds.

The change of the electric power supplied from the power supply apparatus 2 to the dissection device 1 corresponding to the determined type of the blood vessel is performed, for example, as explained below.

First, it is difficult to seal the vein compared with the artery. Therefore, when it is determined that the target blood vessel is the vein, the light housing 3 automatically sets the power supply apparatus 2 such that the electric power supplied from the power supply apparatus 2 to the dissection device 1 is increased to enable the dissection device 1 to exhibit a higher sealing ability (the vein can be more surely sealed). Consequently, it is possible to perform effective treatment without depending on determination by the surgeon (see the explanation of step S9 in Fig. 3 related to the arrows A6 and A7 shown in Fig. 1).

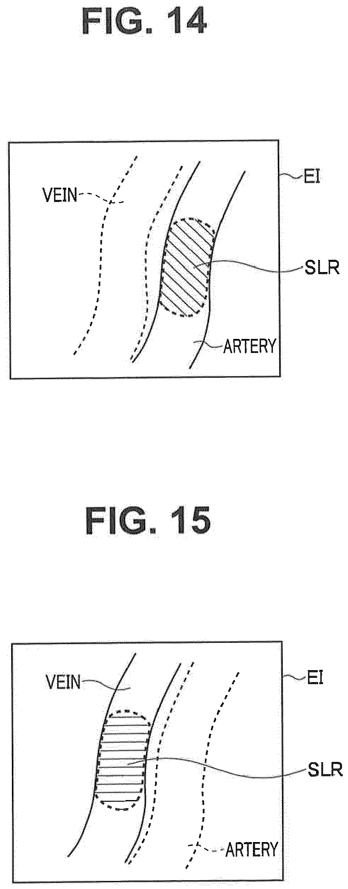

Further, the irradiation of the guide light corresponding to the determined type of the blood vessel is performed, for example, as shown in Fig. 14 and Fig. 15. Fig. 14 is a diagram showing an example of monitor display at a time when the artery (or the parallel running of the artery and the vein) is detected, and for example, the green guide light is irradiated on the subject from the laser light source for guide 32. Fig. 15 a diagram showing an example of monitor display at a time when the vein is detected and the blue guide light is irradiated on the subject from the laser light source for guide 32.

As shown in an endoscopic image EI in Fig. 14, when the artery (or the parallel running of the artery and the vein) is detected, for example, the green (or red to magenta (Mg)) guide light (as a color of the guide light, a color having a tone not masked by a subject color is used) is irradiated on the subject from the laser light source for guide 32 (Fig. 14). Consequently, an irradiation region SLR of the laser light for sensing is observed as a green (or red to magenta (Mg)) region in the endoscopic image EI.

On the other hand, as shown in the endoscopic image EI in Fig. 15, when the vein is detected, the blue guide light (as a color of the guide light, like the green guide light, a color having a tone not masked by the subject color is used) is irradiated on the subject from the laser light source for guide 32 (Fig. 15). Consequently, the irradiation region SLR of the laser light for sensing is observed as a blue region in the endoscopic image EI.

In this way, the color coding display corresponding to the determined type of the blood vessel is performed.

Note that the color coding is performed according to whether the target blood vessel is the artery (or the parallel running of the artery and the vein) or the vein. However, color coding corresponding to a type of a blood vessel may be performed by, for example, changing the light emission ratio of the green laser light source and the blue laser light source in the laser light source for guide 32. As a specific example, the green guide light is irradiated on the artery, the bluish green guide light is irradiated on the parallel running of the artery and the vein, and the blue guide light is irradiated on the vein to perform the color coding display.

### [Determination of depth of the blood vessel]

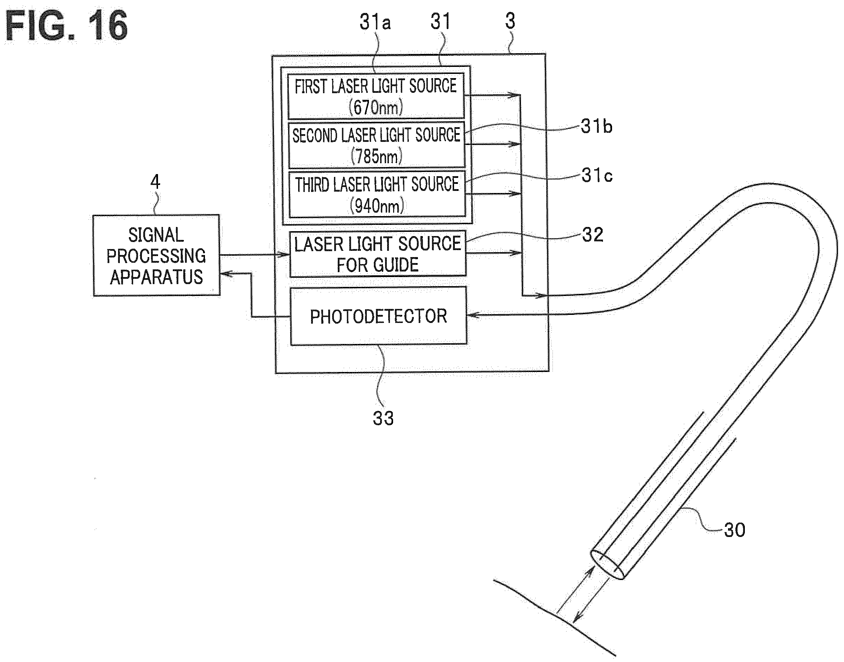

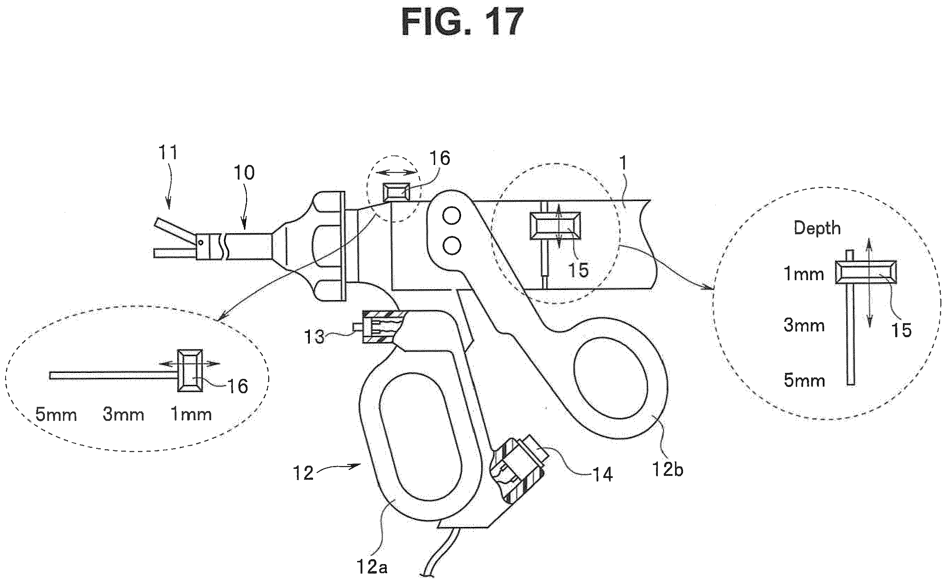

Determination of depth of the blood vessel is explained with reference to Fig. 16 and Fig. 17.

Incidentally, when there is 3D information of the blood vessel inside the tissue, it is easy to decide a treatment policy concerning how dissection should be performed. Therefore, it is useful to determine a rough depth position (running pattern) of the blood vessel from a tissue surface using a laser Doppler measurement technique.

Therefore, for example, the wavelength of the laser light for sensing is changed to multiple wavelengths and detection of a blood vessel depth is performed based on reflected light intensity of lights having the respective wavelengths (however, this does not preclude the detection of the blood vessel depth from being performed using the laser light for sensing having a single wavelength).

Fig. 16 is a diagram showing a configuration example in which a laser light source of a three-wavelength irradiation type is used as the laser light source for blood vessel sensing 31.

In the example shown in Fig. 16, the laser light source for blood vessel sensing 31 includes, for example, three kinds of semiconductor laser light sources, that is, a first laser light source 31a configured to emit laser light for sensing of 670 nm, a second laser light source 31b configured to emit laser light for sensing of 785 nm, and a third laser light source 31 c configured to emit laser light for sensing of 940 nm. All of the laser light sources 31 a, 31b, and 31c and the laser light source for guide 32 irradiate the laser lights on the subject via the optical fiber 30. Return lights are detected by the photodetector 33 via the optical fiber 30.

In general, a reaching degree of light to depth of the tissue is higher as a wavelength of the light is longer. Therefore, when Doppler signals having high reflected light intensity are obtained at all of 670 nm, 785 nm, and 940 nm, it is determined that the blood vessel is present in a relatively shallow portion of a living organism.

When a Doppler signal having low reflected light intensity is obtained at 670 nm and Doppler signals having high reflected light intensity are obtained at 785 nm and 940 nm, it is determined that the blood vessel is present at intermediate depth of the living organism.

Further, when a signal having high reflected light intensity is obtained at only 940 nm, it is determined that the blood vessel is present in the deep part of the living organism.

The color of the guide light irradiated from the laser light source for guide 32 is changed according to a determination result of the depth of the blood vessel. For example, the color of the guide light is changed in such a manner that, when the blood vessel is present in a shallow place, the red to magenta (Mg) guide light is irradiated, when the blood vessel is present at intermediate depth, the green guide light is irradiated, and when the blood vessel is present in a deep place, the blue guide light is irradiated.

Note that, for example, the light emission ratio of the green laser light source and the blue laser light source in the laser light source for guide 32 may be changed to perform color coding display corresponding to the depth of the blood vessel. As a specific example, the green guide light is irradiated on a shallow blood vessel, the bluish green guide light is irradiated on a blood vessel at intermediate depth, and the blue guide light is irradiated on a deep blood vessel to perform color coding display.

Fig. 17 is a diagram showing an example in which operation members for setting depth of the target blood vessel are disposed near the handle section 12 of the dissection device 1.

Concerning the determination of the depth of the target blood vessel, for example, the first slide section 15 and the second slide section 16 are used as the operation members for setting the thresholds of the characteristics of the target blood vessel. The first button section 13 and the second button section 14 are used for trigger signal generation for power application by the power supply apparatus 2 (however, at least one of the first button section 13 and the second button section 14 may be used as the operation member for setting the thresholds of the characteristics of the target blood vessel). Therefore, as the operation member for setting the thresholds of the characteristics of the target blood vessel, both of the first slide section 15 and the second slide section 16 do not need to be provided. At least one of the first slide section 15 and the second slide section 16 only has to be provided.

In this case, for example, in the first slide section 15 and the second slide section 16, indicators "1 mm", "3 mm", and "5 mm" indicating depths of blood vessels are described together with characters "Depth" according to necessity. For example, the medical apparatus is set such that the first slide section 15 is slid up and down with the thumb of the surgeon (or the second slide section 16 is slid back and forth with the index finger of the surgeon) and aligned with a position of any one of the indicators, whereby a blood vessel having thickness designated by the indicator (or a blood vessel having thickness equal to or larger than the thickness (a threshold of thickness) designated by the indicator) is sensed.

Note that the indicators indicating the depth of the blood vessel are not limited to the numbers indicating the depths described above. Characters such as "Deep", "Middle", and "Shallow" may be used.

Therefore, the first slide section 15 and the second slide section 16 function as a threshold switching section disposed in the handle section 12, which is the operation section, and capable of changing a threshold of depth information of a blood vessel acquired by the information acquiring section.

When the guide light is irradiated by the laser light source for guide 32 on the blood vessel, the depth of which is detected as explained above, to perform the guide display, the guide display is performed, for example, as explained below.

When a blood vessel detected based on blood flow velocity is present deep (e.g., depth≥4 mm), the green guide light is irradiated on the subject from the laser light source. On the other hand, when the blood vessel is present in a relatively shallow region (e.g., depth<4 mm), the blue guide light is irradiated on the subject from the laser light source.

Further, when the surgeon desires to detect a deeper blood vessel, the medical apparatus is set such that the surgeon operates the handle section 12 and the like to generate a trigger signal, whereby the laser light for sensing is emitted from a laser light source having a longer wavelength (e.g., an LD light source of 940 nm) incorporated in the laser light source for blood vessel sensing 31.

### [Method of displaying detected blood vessel using the guide light]

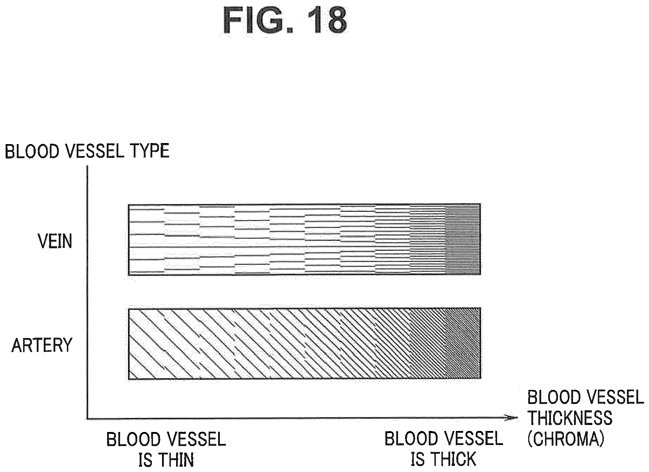

A method of displaying a detected blood vessel using the guide light is explained with reference to Fig. 18 to Fig. 21.

The characteristics of the blood vessel detected as explained above, that is, the thickness of the blood vessel, the type (the artery/the vein) of the blood vessel, and the depth of the blood vessel are specified and displayed as shown in Fig. 18 to Fig. 21 below using the guide light.

First, Fig. 18 is a diagram showing an example in which the green guide light is irradiated on the artery, the blue guide light is irradiated on the vein, and chroma of blue or green is set higher as the thickness of the blood vessel is larger.

Light desirably used as the guide light is light in a wavelength band with low reflectance in a living organism (a parenchyma or a fat tissue), more specifically, blue to green light. Therefore, the green guide light is irradiated on the artery and the blue guide light is irradiated on the vein (at this point, as explained above, the light emission ratio of the green light and the blue light may be changed to color-code and display three patterns of "running as the artery alone", "running as the vein alone", and "parallel running of the artery and the vein"). Consequently, when the surgeon observes the blood vessel on which the guide light is irradiated, for example, the surgeon can understand that the blood vessel is the artery if the guide light is green and understand that the blood vessel is the vein if the guide light is blue (or understand that the blood vessel is the parallel running of the artery and the vein if the guide light is bluish green).

At this point, further, it is desirable to change chroma of the guide light according to the thickness of the blood vessel as shown in Fig. 18. The change of the chroma of the guide light is performed by, for example, changing light emission intensity of the laser light source. As a specific application example, it is desirable to set the chroma of the guide light higher as the blood vessel is thicker. Therefore, the guide-light-emission-signal transmitting section 43 controls the light emitting section to change energy density of the notification light having the first or second wavelength according to a degree of the first or second characteristic. Consequently, when the surgeon observes the blood vessel on which the guide light is irradiated, the surgeon can recognize thickness of the artery or the vein according to the chroma.

In addition to the thickness of the blood vessel and the type of the blood vessel, it is desirable to enable the surgeon to recognize depth of the blood vessel as well using the guide light. For example, normal guide light is irradiated when the blood vessel is deep (e.g., when the blood vessel is deeper than 4 mm) and the guide light is flashed and irradiated (i.e., as pulse light). Further, flashing velocity (a pulse cycle) may be changed according to the depth. For example, the flashing velocity is set higher as the blood vessel is deeper (when the blood vessel is present deep, since the surgeon cannot visually recognize the presence of the blood vessel, laser is flashed to call attention of the surgeon). In this case, the guide-light-emission-signal transmitting section 43 controls the light emitting section to make the notification light having the first or second wavelength the pulse light and change the pulse cycle according to the degree of the first or second characteristic.

A spot size of the guide light is larger than a spot size of the laser light for sensing. As a specific example, the spot size of the guide light is, for example, 3.0 mm in diameter. The spot size of the laser light for sensing is 0.3 mm in diameter.

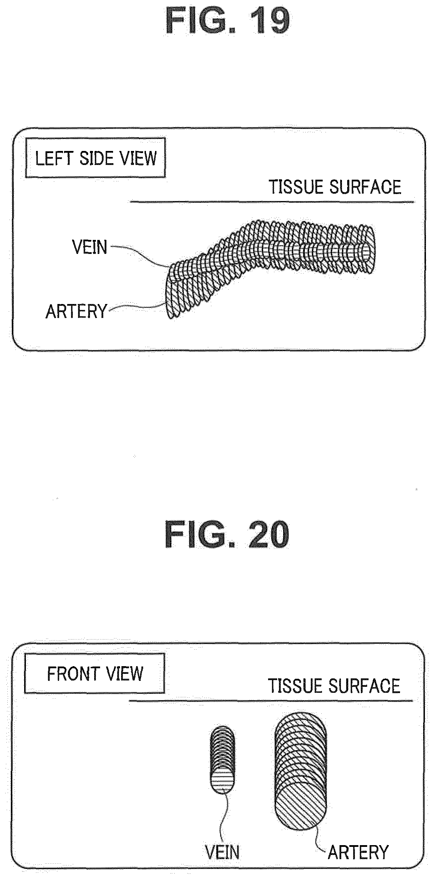

It is desirable to perform, for example, stereoscopic display (3D display) shown in Fig. 19 to Fig. 21 by performing image processing of images, which are obtained by performing spot irradiation of the guide light a plurality of times, and combining the images.

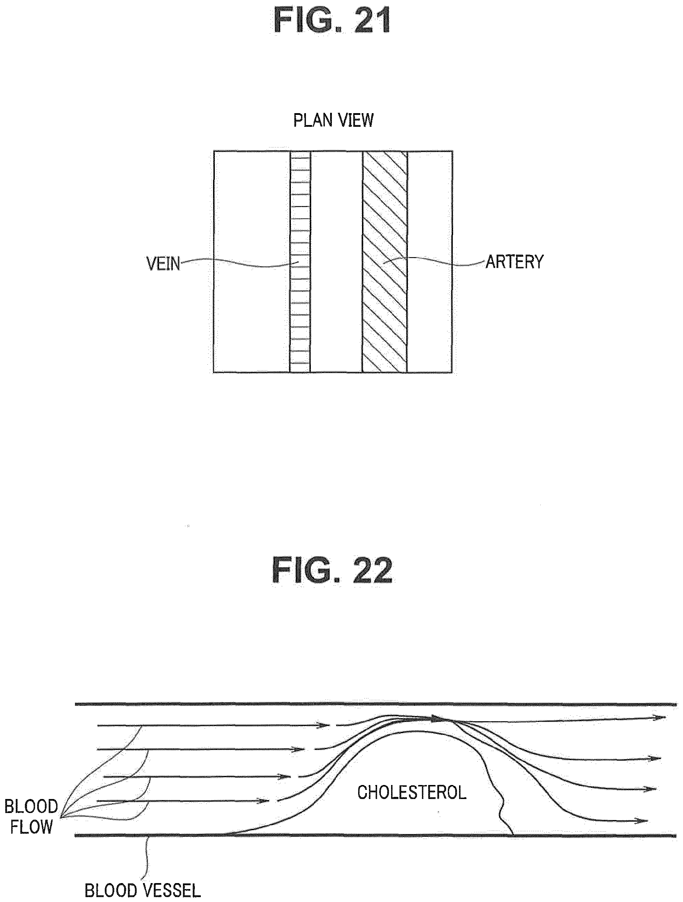

Fig. 19 is a left side view showing an example in which images obtained by irradiating the green guide light a plurality of times along the artery and irradiating the blue guide light a plurality of times along the vein are superimposed and the artery and the vein are displayed. Fig. 20 is a front view showing the example in which the images obtained by irradiating the green guide light a plurality of times along the artery and irradiating the blue guide light a plurality of times along the vein are superimposed and the artery and the vein are displayed. Fig. 21 is a plan view showing the example in which the images obtained by irradiating the green guide light a plurality of times along the artery and irradiating the blue guide light a plurality of times along the vein are superimposed and the artery and the vein are displayed.

Fig. 19 to Fig. 21 show, using three-view drawings, an example in which the blood vessel is displayed stereoscopically (as pseudo 3D). Among the three-view drawings, if the surgeon views the left side view shown in Fig. 19 and the front view shown in Fig. 20, the surgeon can know in which degree of depth from a tissue surface the blood vessel is running and what kind of change occurs in a depth direction. If the surgeon views the front view shown in Fig. 20 and the plan view shown in Fig. 21, the surgeon can know at which interval the artery and the vein run in parallel. Further, the surgeon can know the thicknesses of the artery and the vein by viewing any one of the figures.

The characteristics of the detected blood vessel are not limited to be guide-displayed using the guide light. Instead of or in addition to the guide display, the characteristics (the type, the thickness, the depth, and the like of the blood vessel) may be displayed on the monitor using characters, figures, and the like. As an example, when it is detected that the type of the blood vessel is the artery as the characteristic of the blood vessel, "Artery" is displayed on the monitor. In this case, the monitor functions as the notifying section. Further, alternatively, the characteristics (the type, the thickness, the depth, and the like of the blood vessel) may be notified by sound or the like. In this case, the medical apparatus includes a speaker or the like that emits sound. The speaker or the like functions as the notifying section.

### [Application example to arteriosclerosis]

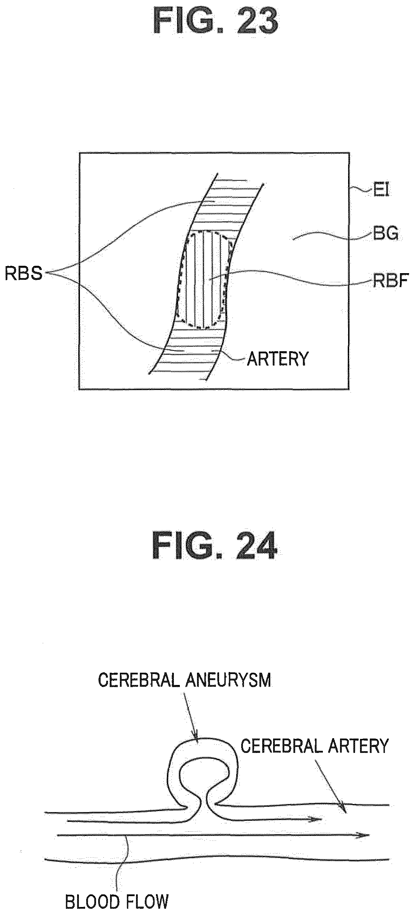

An application example to arteriosclerosis is explained with reference to Fig. 22 and Fig. 23.

First, Fig. 22 is a diagram showing an example in which a region of arteriosclerosis emerges in the blood vessel because of cholesterol or the like and flow velocity of a blood flow changes.

For example, when cholesterol or the like is accumulated in the artery, arteriosclerosis sometimes occurs. In such a region of the arteriosclerosis, since a lumen is locally constricted, as shown in Fig. 22, the blood flow velocity is high in an arteriosclerosis portion compared with a periphery where arteriosclerosis does not occur. Therefore, if the laser Doppler detection is performed to measure the blood flow velocity, it is possible to find the arteriosclerosis region.

Therefore, blood flow velocity of the artery is detected on a real-time basis and guide light having a color corresponding to the detected blood flow velocity is irradiated on the artery on a real-time basis as shown in Fig. 23. Fig. 23 is a diagram showing an example in which a portion RBF (the arteriosclerosis portion) where the blood flow is relatively fast and a portion RBS (a portion other than the arteriosclerosis) where the blood flow is relatively slow are color-coded by guide lights having different colors.

Specific examples of the color-coding include irradiating the red to magenta (Mg) or green guide light on the portion RBF corresponding to the arteriosclerosis and irradiating the blue guide light on the portion RBS other than the arteriosclerosis of the artery. As a color of the guide light, a color having a tone not masked by the subject color is used. Note that the guide light is not irradiated on the region other than the blood vessel (a background tissue BG). Therefore, the region is displayed in a tone of a normal light observation under an observation by an endoscope such as a laparoscope.

Consequently, by observing the endoscopic image EI, it is possible to recognize, on a real-time basis, the region where the arteriosclerosis occurs and it is possible to perform early finding of arterial sclerosis.

### [Application example to cerebral aneurysm]

An application example to cerebral aneurysm is explained with reference to Fig. 24 to Fig. 31.

First, Fig. 24 is a diagram showing a state in which cerebral aneurysm occurs in a cerebral artery and a blood flow occurs in the cerebral aneurysm.

When the cerebral aneurysm occurs in the cerebral artery, a blood flow changes compared with a peripheral cerebral artery, for example, the blood flow is held up in the cerebral aneurysm. Therefore, if the laser Doppler detection is performed to measure blood flow velocity, it is possible to find the cerebral aneurysm.

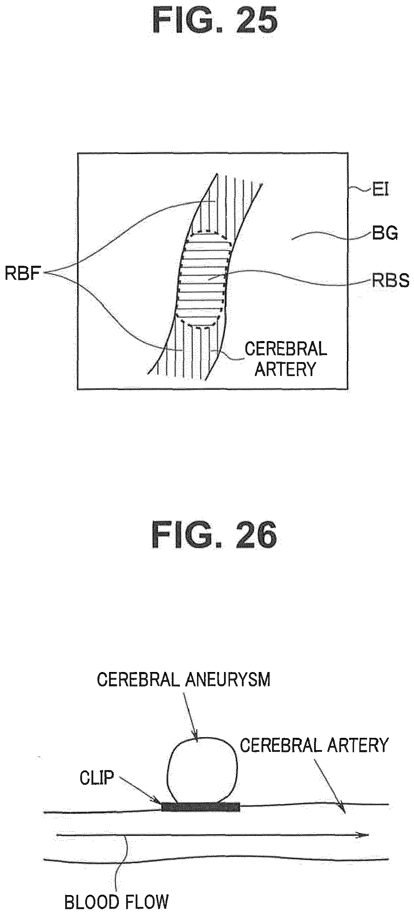

Therefore, blood flow velocity of the cerebral artery is detected on a real-time basis. Guide light having a color corresponding to the detected blood flow velocity is irradiated on the cerebral artery on a real-time basis as shown in Fig. 25. Fig. 25 is a diagram showing an example in which the portion RBS (a cerebral aneurysm portion) where the blood flow is relatively slow and the portion RBF (a portion other than the cerebral aneurysm) where the blood flow is relatively fast are color-coded by guide lights having different colors.

Specific examples of the color-coding include irradiating the blue guide light on the portion RBS where the blood flow is relatively slow corresponding to the cerebral aneurysm and irradiating the red to magenta (Mg) or green guide light on the portion RBF where the blood flow is relatively fast corresponding to the cerebral artery other than the cerebral aneurysm. As a color of the guide light, a color having a tone not masked by a subject color is used.

Consequently, by observing the endoscopic image EI, it is possible to recognize, on a real-time basis, a region where the cerebral aneurysm occurs.

The measurement of the blood flow velocity by the laser Doppler detection and the irradiation of the guide light corresponding to the blood flow velocity can also be used to determine an effect of cerebral aneurysm treatment.

Fig. 26 is a diagram showing a state in which clipping treatment is performed on the cerebral aneurysm and the blood flow in the cerebral aneurysm is stopped.

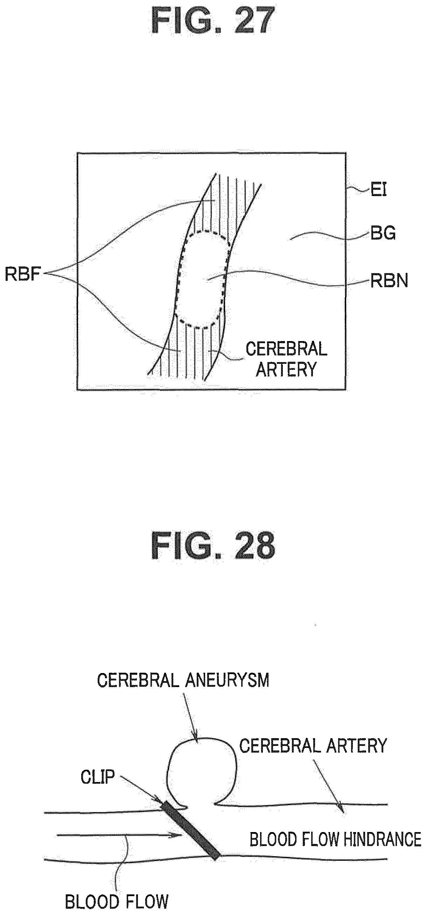

When the cerebral aneurysm treatment by a clip (clipping treatment) is successful, the blood flow in the cerebral aneurysm is stopped but there is no change in the blood flow of the cerebral artery. Therefore, as shown in the endoscopic image EI in Fig. 27, the guide light is not irradiated on a portion RBN where the blood flow is stopped corresponding to the cerebral aneurysm (i.e., the portion RBN is displayed in the tone of the normal light observation). The red to magenta (Mg) or green illumination light is irradiated on the portion RBF of the cerebral artery where the blood flow is relatively fast. Fig. 27 is a diagram showing an example of color-coding by the guide lights of the portion RBN (the cerebral aneurysm portion) where the blood flow is stopped by the clipping treatment and the portion RBF (the portion other than the cerebral aneurysm) where the blood flow is relatively fast.

Therefore, after the surgeon performs the clipping treatment on the portion RBS of the cerebral aneurysm shown in Fig. 25, when the endoscopic image EI shown in Fig. 27 is observed, the surgeon can recognize that the clipping treatment is successful and end medical treatment.

Fig. 28 is a diagram showing a state in which the clipping treatment is performed on the cerebral artery other than the cerebral aneurysm and a portion where a blood flow is hindered emerges in the cerebral artery.

When the clipping is performed on, for example, the cerebral artery rather than being performed on only the cerebral aneurysm, a state in which the blood flow of the cerebral artery is hindered occurs.

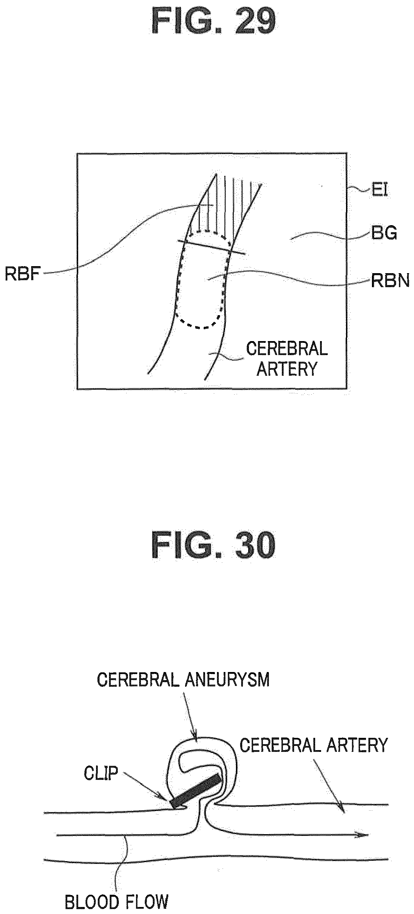

In this case, whereas the guide display is as shown in Fig. 25 before the clipping treatment, after the clipping treatment, the guide display changes to guide display in which the guide light is not irradiated on part of or the entire artery centering on a clip position in an observation range as shown in Fig. 29. Fig. 29 is a diagram showing an example of color-coding by the guide lights of the portion RBN (the cerebral aneurysm and the cerebral artery in which the blood flow is hindered) where the blood flow is stopped by the clipping treatment and the portion RBF (the cerebral artery in which the blood flow is not hindered) where the blood flow is relatively fast.

Therefore, after the surgeon performs the clipping treatment on the portion RBS of the cerebral aneurysm shown in Fig. 25, when the endoscopic image EI shown in Fig 29 is observed, the surgeon only has to determine that the clipping treatment is unsuccessful and perform the clipping treatment again.

Further, Fig. 30 is a diagram showing a state in which the blood flow remains in the cerebral aneurysm because the clipping treatment on the cerebral aneurysm is insufficient.

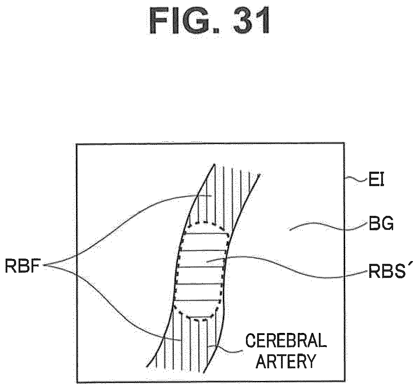

When the clipping treatment on the cerebral aneurysm is insufficient and the blood flow remains in the cerebral aneurysm, as shown in a portion RBS' of the cerebral aneurysm in Fig. 31, the guide light remains being irradiated (however, when the chroma is changed according to the blood flow velocity, the chroma is sometimes different). Fig. 31 is a diagram showing an example of color-coding by the guide lights of the portion RBS' (the cerebral aneurysm portion) where the slow blood flow remains because of the insufficient clipping treatment and the portion RBF (the portion other than the cerebral aneurysm) where the blood flow is relatively fast.

Therefore, after the surgeon performs the clipping treatment on the portion RBS of the cerebral aneurysm shown in Fig. 25, when the endoscopic image EI shown in Fig. 31 is observed, the surgeon only has to determine that the clipping treatment is insufficient and perform the clipping treatment again.

Note that cerebral aneurysm is conventionally detected by ultrasound Doppler. However, an ultrasound probe needs to be brought into contact with a subject to transmit ultrasound to the subject. On the other hand, in the present technique for detecting the cerebral aneurysm with the laser Doppler, since the laser light for sensing is used, it is possible to feed light into the subject in a noncontact manner and observe the subject. The present technique is superior in operability to the conventional technique in which the ultrasound is used.

### [Laser light for sensing]

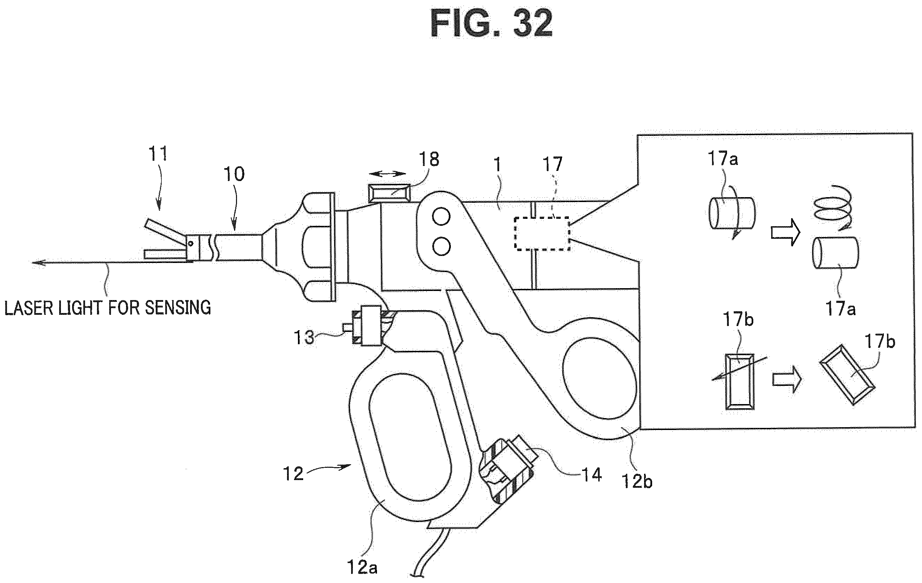

Emitting operation and an irradiation direction of the laser light for sensing are explained with reference to Fig. 32 to Fig. 34.

First, Fig. 32 is a diagram showing an example of disposition of operation members for emitting the laser light for sensing near the handle section of the dissection device.

The first button section 13 is provided on the inserting direction side of the proximal end portion of the first handle 12a in the handle section 12. The second button section 14 is provided on the second handle 12b side of the distal end portion of the first handle 12a. A first operation section 17 is provided on the side surface further on the hand side than the second handle 12b of the main body that supports the first handle 12a and the second handle 12b. A second operation section 18 is provided on the upper surface of the main body that supports the first handle 12a and the second handle 12b. The first operation section 17 is configured as a dial section 17a of a rotating operation type, a pushing section 17b of a pressing operation type, or the like. The second operation section 18 is configured as, for example, a pushing section of the pressing operation type.

By operating any one of the dial section 17a, the pushing section 17b, the second operation section 18, and the first button section 13 shown in Fig. 32, the laser light for sensing is configured to be emitted from the laser light source for blood vessel sensing 31. Note that the laser light for sensing may be emitted from the laser light source for blood vessel sensing 31 as explained above when the surgeon steps on a footswitch.

The laser light for sensing (or the guide light) irradiated from the distal end of the optical fiber 30 may be outputted in parallel to an axial direction of the insertion section 10 of the dissection device 1 shown in Fig. 33. Alternatively, as shown in Fig. 34, a light reflecting object 19a may be disposed in the lower jaw 11b configuring the treatment section 11 functioning as a grasping section and a photodetector 19b may be disposed in the upper jaw 11a to transmit the laser light for sensing (or the guide light) from the lower jaw 11b to the upper jaw 11a and receive the laser light for sensing (or the guide light).

Fig. 33 is a diagram showing an example in which the laser light for sensing is outputted in the axial direction from the distal end portion of the dissection device. Fig. 34 is a diagram showing an example in which the laser light for sensing is outputted from the lower jaw toward the upper jaw of the distal end portion of the dissection device.

According to the embodiment explained above, the more detailed information concerning the blood vessel, that is, the information concerning not only the presence of the blood vessel but also the thickness and the depth of the blood vessel and the artery/the vein is provided to the surgeon on a real-time basis. Therefore, it is possible to improve safety and simplicity of medical services such as a laparoscopic operation.

In this case, when the laser light for guide corresponding to the characteristics of the blood vessel is irradiated, in an endoscopic image, it is possible to visually recognize which blood vessel has what kinds of characteristics. Therefore, convenience for the surgeon is high. It is possible to further improve the safety.

Further, the acquired information is fed back to the power supply apparatus 2 to perform optimum output setting of the dissection device 1. Therefore, it is possible to further improve the safety and the simplicity of the medical services such as the laparoscopic operation.

For example, it is possible to not only inform, while the surgeon is performing the laparoscopic operation, the surgeon on a real-time basis that a blood vessel hard to be viewed is present but also to inform characteristics of the blood vessel (a type, thickness, depth, and the like of the blood vessel) on a real-time basis. Therefore, a frequency of intraoperative bleeding can be reduced. It is possible to perform a safe laparoscopic operation.

It is possible to distinguish, for example, a cystic artery and a cystic duct by whether there is a pulsation. Alternatively, it is possible to more easily find a middle colic artery that is hard to be found because there is variation in running.

Thickness of a blood vessel to be detected can be changed by the operation section of the handle section 12. Therefore, it is possible to perform adjustment not to set a capillary as a detection target. Such a function of changing thresholds achieves a profound effect on a lever and a spleen including a lot of capillaries.

The power supply apparatus 2 is feedback-controlled based on the characteristics of the blood vessel specified by the blood-vessel-characteristic determination circuit 42. Therefore, it is possible to optimize an output of the power supply apparatus 2 without requiring manual setting of the surgeon.

Note that the respective sections explained above may be configured as circuits. Any circuit may be implemented as a single circuit or may be implemented as a circuit obtained by combining a plurality of circuits as long as the circuit can play the same function. Further, any circuit is not limited to a circuit configured as a dedicated circuit for performing a target function and may be configured to perform the target function by causing a general-purpose circuit to execute a processing program.

The medical apparatus is mainly explained above. However, the present invention may be an operating method for the medical apparatus for operating the medical apparatus as explained above or may be a processing program for causing a computer to perform processing the same as the processing of the medical apparatus, a computer-readable non-transitory recording medium having the processing program recorded therein, and the like.

Further, the present invention is not limited to the embodiment per se. In an implementation stage, the constituent elements can be modified and embodied in a range not departing from the spirit of the present invention. Aspects of various inventions can be formed by appropriate combinations of the plurality of constituent elements disclosed in the embodiment. For example, several constituent elements can be deleted from all the constituent elements described in the embodiment. Further, the constituent elements described in different embodiments may be combined as appropriate. In this way, it goes without saying that various modifications and applications are possible in a range not departing from the spirit of the invention.

### [Notes]

According to the embodiment of the present invention explained in detail above, configurations explained below can be obtained.
(Note item A1) A medical apparatus including:
   a treatment device including an operation section configured to perform operation for dissecting a tissue of a subject;
   an illuminating section configured to irradiate illumination light for detecting a blood vessel of the subject;
   an information acquiring section configured to detect a spectrum of return light from the subject of the illumination light irradiated by the illuminating section and acquire, based on the spectrum, thickness information of the blood vessel serving as a characteristic of the blood vessel in an irradiation region where the illumination light is irradiated on the subject; and
   a threshold switching section disposed in the operation section and capable of changing a threshold of the thickness information of the blood vessel acquired by the information acquiring section.
(Note item A2) A medical apparatus including:
   a treatment device including an operation section configured to perform operation for dissecting a tissue of a subject;
   an illuminating section configured to irradiate illumination light for detecting a blood vessel of the subject;
   an information acquiring section configured to detect a spectrum of return light from the subject of the illumination light irradiated by the illuminating section and acquire, based on the spectrum, depth information of the blood vessel serving as a characteristic of the blood vessel in an irradiation region where the illumination light is irradiated on the subject; and
   a threshold switching section disposed in the operation section and capable of changing a threshold of the depth information of the blood vessel acquired by the information acquiring section.
(Note item A3) A medical apparatus including:
   a treatment device including an operation section configured to perform operation for dissecting a tissue of a subject;
   an illuminating section configured to irradiate illumination light for detecting a blood vessel of the subject;
   an information acquiring section configured to detect a spectrum of return light from the subject of the illumination light irradiated by the illuminating section and acquire, based on the spectrum, type information of the blood vessel serving as a characteristic of the blood vessel indicating whether the blood vessel in an irradiation region where the illumination light is irradiated on the subject is an artery or a vein; and
   a threshold switching section disposed in the operation section and capable of changing a threshold of the type information of the blood vessel acquired by the information acquiring section.
(Note item A4) A medical apparatus including:
   a treatment device including an operation section configured to perform operation for dissecting a tissue of a subject;
   an illuminating section configured to irradiate illumination light for detecting a blood vessel of the subject;
   an information acquiring section configured to detect a spectrum of return light from the subject of the illumination light irradiated by the illuminating section and acquire, based on the spectrum, as a characteristic of the blood vessel in an irradiation region where the illumination light is irradiated on the subject, at least one of thickness information of the blood vessel, depth information of the blood vessel, and type information of the blood vessel indicating whether the blood vessel is an artery or a vein; and
   a threshold switching section disposed in the operation section and capable of changing a threshold of the characteristic of the blood vessel acquired by the information acquiring section, wherein
   the threshold switching section switches the characteristic of the blood vessel acquired by the information acquiring section from a first characteristic including at least one characteristic of the thickness information of the blood vessel, the depth information of the blood vessel, and the type information of the blood vessel to a second characteristic including at least one characteristic of the thickness information of the blood vessel, the depth information of the blood vessel, and the type information of the blood vessel, the second characteristic being not completely the same as the first characteristic.
(Note item A5) The processing device according to any one of the note items A1 to A4, wherein
   the operation section includes a handle section to be held by a hand that operates the treatment device, and
   the threshold switching section is provided in a position where the threshold switching section is operable by the hand that holds the handle section.
(Note item B1) A processor including:
   an input section to which information indicating a frequency spectrum of light from a subject including a blood vessel is inputted;
   a fluctuation monitoring section configured to monitor temporal fluctuation of information indicating the frequency spectrum inputted to the input section and, when the temporal fluctuation exceeds a predetermined threshold, detect the temporal fluctuation as an excess over the threshold; and
   an output section configured to, when the excess over the threshold is detected by the fluctuation monitoring section, output information indicating that the blood vessel is an artery and, when the excess over the threshold is not detected, output information indicating that the blood vessel is a vein.

This application is filed claiming priority from Japanese Patent Application No. 2015-86977 filed in Japan on April 21, 2015, disclosed contents of which are incorporated in the present specification, claims, and drawings by reference.

## Claims

1. A medical apparatus comprising:
an illuminating section connected to an insertion object, which is inserted into a body cavity of a subject, and configured to irradiate illumination light for detecting a blood vessel of the subject;
a detecting section configured to detect return light from the body cavity of the subject of the illumination light irradiated by the illuminating section;
a calculating section configured to calculate, based on a detection result of the detecting section, at least one of information concerning scattering of blood cells in the blood vessel and information concerning absorption by blood in the blood vessel;
a determining section configured to determine a characteristic of the blood vessel based on a calculation result of the calculating section; and
a notifying section configured to perform notification based on the characteristic of the blood vessel determined by the determining section.

2. The medical apparatus according to claim 1, wherein
when the calculating section calculates the information concerning the scattering, the calculating section analyzes a frequency spectrum of the return light and calculates, from the frequency spectrum, as the information concerning the scattering, a parameter concerning flowing velocity of blood cells in the blood vessel, and
the determining section determines, based on the parameter calculated by the calculating section, at least one of thickness of the blood vessel, whether the blood vessel is an artery or a vein, and depth of the blood vessel in the subject as the characteristic of the blood vessel.

3. The medical apparatus according to claim 2, wherein
the determining section determines the characteristic of the blood vessel in plurality, and
the notifying section performs notification in a plurality of notification forms in combination according to a combination of the characteristic of the blood vessel in plurality determined by the determining section.

4. The medical apparatus according to claim 2, wherein the notifying section includes:
a light emitting section capable of selectively generating notification light having a first wavelength to be irradiated on an irradiation near region including an irradiation region on which the illumination light is irradiated in the subject and notification light having a second wavelength different from the first wavelength to be irradiated on the irradiation near region; and
a control section configured to control the light emitting section to emit the notification light having the first wavelength as a first notification method when the characteristic of the blood vessel determined by the determining section is a first characteristic and control the light emitting section to emit the notification light having the second wavelength as a second notification method different from the first notification method when the characteristic of the blood vessel determined by the determining section is a second characteristic different from the first characteristic.

5. The medical apparatus according to claim 4, wherein the control section controls, according to a degree of the first or second characteristic, the light emitting section to change energy density of the notification light having the first or second wavelength.

6. The medical apparatus according to claim 4, wherein the control section controls, according to a degree of the first or second characteristic, the light emitting section to make the notification light having the first or second wavelength pulse light and change a pulse cycle.

7. The medical apparatus according to claim 4, wherein the light emitting section generates blue light as the notification light having the first wavelength and generates green light as the notification light having the second wavelength.

8. The medical apparatus according to claim 1, wherein
the illuminating section includes a laser light source configured to generate laser light as the illumination light, and
the medical apparatus further comprises a treatment device capable of dissecting a tissue of the subject.

9. The medical apparatus according to claim 1, further comprising:
a treatment device capable of operating in a plurality of operation modes in which blood vessel sealing abilities are different; and
a control section configured to switch the operation modes of the treatment device according to the characteristic of the blood vessel determined by the determining section.

10. The medical apparatus according to claim 2, wherein the calculating section calculates, as the parameter concerning the flowing velocity of the blood cells in the blood vessel, an integrated value of intensity of the frequency spectrum, a gradient of the frequency spectrum, or an average frequency of the frequency spectrum.

11. An operating method for a medical apparatus comprising:
a step in which an illuminating section connected to an insertion object, which is inserted into a body cavity of a subject, irradiates illumination light for detecting a blood vessel of the subject;
a step in which a detecting section detects return light from the body cavity of the subject of the illumination light irradiated by the illuminating section;
a step in which a calculating section calculates, based on a detection result of the detecting section, at least one of information concerning scattering of blood cells in the blood vessel and information concerning absorption by blood in the blood vessel;
a step in which a determining section determines a characteristic of the blood vessel based on a calculation result of the calculating section; and
a step in which a notifying section performs notification based on the characteristic of the blood vessel determined by the determining section.
